# EUROPEAN PATENT APPLICATION

(11) **EP 1 787 866 A1**
(43) Date of publication of application: **23.05.2007**
(21) Application number: 05765532.6
(22) Date of filing: 11.07.2005
(51) Int. Cl.: B60R 11/02, B60R 16/02

(54) **INFORMING DEVICE**

(30) Priority: 14.07.2004 JP 2004207554
(71) Applicant: Matsushita Electric Industrial Co., Ltd., Kadoma-shi, Osaka 571-8501 (JP)
(72) Inventor: HAMADA, Hiroyuki c/o Matsuhita El. Ind. Co., Ltd., Osaka-shi, Osaka 540-6319 (JP); IBARAKI, Susumu c/o Matsuhita El. Ind. Co., Ltd., Osaka-shi, Osaka 540-6319 (JP); MORIMOTO, Akihiro c/o Matsuhita El. Ind. Co., Ltd., Osaka-shi, Osaka 540-6319 (JP); OTSUKA, Masakuzu c/o Matsuhita El. Ind. Co., Ltd., Osaka-shi, Osaka 540-6319 (JP); KUWABARA, Takashi c/o Matsuhita El. Ind. Co., Ltd., Osaka-shi, Osaka 540-6319 (JP); NAKAGAWA, Seiichi c/o Matsuhita El. Ind. Co., Ltd., Osaka-shi, Osaka 540-6319 (JP); NAGASHIMA, Kazumasa c/o Matsuhita E. In. Co., Ltd., Osaka-shi, Osaka 540-6319 (JP)
(74) Representative: Pautex Schneider, Nicole Véronique
(86) International application number: PCT/JP2005/012742
(87) International publication number: WO 2006/006553

(57) **Abstract**

The motion sickness preventing apparatus **10** comprises acceleration detecting means **11** for detecting an acceleration of a vehicle, navigation means **12** for outputting support information to support a driver in driving to a destination through an optimum travel route, image taking means **13** for taking an image of an object near or ahead of the vehicle, motion detecting means **14** for detecting a motion of the vehicle, image and sound producing means **15** for producing an image signal and a sound signal, image displaying means **16** for displaying an image in response to the image signal produced by the image and sound producing means **15,** sound outputting means **17** for outputting a sound in response to the sound signal produced by the image and sound producing means **15,** and controlling means **18** for controlling the image displaying means **16** and the sound outputting means **17** on the basis of the motion of the vehicle detected by the motion detecting means **14.** The motion sickness preventing apparatus **10** can enhance an effect of allowing vehicle occupants to enjoy comfortable driving without allowing them to observe the motion of the vehicle with their eyes.

## Description

### TECHNICAL FIELD OF THE INVENTION

The present invention relates to an informing device for enhancing an effect of allowing vehicle occupants to enjoy comfortable driving.

### DESCRIPTION OF THE RELATED ART

As an apparatus helpful in allowing vehicle occupants to enjoy comfortable driving, there have been known a wide variety of display apparatuses, one typical example of which is disclosed in patent document 1, and adapted to prevent the vehicle occupants from having motion sickness by comprising an indicating apparatus for indicating whether or not the vehicle is about to turn to the right, or the left, whether or not the driver is about to accelerate, or decelerate the vehicle, and whether or not the driver stops the vehicle.
patent document 1: Jpn. unexamined patent publication No. 2002-154350 (p. 2, FIG. 1)

### DISCLOSURE OF THE INVENTION

### PROBLEMS TO BE SOLVED BY THE INVENTION

The conventional informing device, however, encounters such a problem that the vehicle occupants tend to have motion sickness by failing to pay attention to the indicated information about whether or not the vehicle is about to turn to the right, or the left, whether or not the driver is about to accelerate, or decelerate the vehicle, and whether or not the driver stops the vehicle.

It is, therefore, an object of the present invention to provide an informing device which can enhance an effect of allowing vehicle occupants to enjoy comfortable driving.

### MEANS FOR SOLVING THE PROBLEMS

The informing device according to the present invention comprises: motion detecting means for detecting a motion of a vehicle; outputting means for outputting a sound; and controlling means for controlling, on the basis of the motion of the vehicle detected by the motion detecting means, the sound to be outputted by the outputting means.

The informing device thus constructed as previously mentioned according to the present invention can inform vehicle occupants about the motion of the vehicle by using the sound produced on the basis of the motion of the vehicle. Accordingly, the vehicle occupants can anticipate a change of the motion of the vehicle without observing carefully a motion of the vehicle with their eyes, and keep pace with the change of the motion of the vehicle. In other words, the informing device according to the present invention can enhance an effect of allowing the vehicle occupants to enjoy comfortable driving without allowing them to observe carefully a motion of the vehicle with their eyes.

In the informing device according to the present invention, the outputting means may be adapted to output a sound with an image.

The informing device thus constructed as previously mentioned according to the present invention can inform the vehicle occupants about the motion of the vehicle, by using a sound produced on the basis of the motion of the vehicle, while allowing the vehicle occupants to watch television and the like.

In the informing device according to the present invention, the controlling means may be adapted to allow the outputting means to synchronize the image with the sound by controlling the outputting means on the basis of the motion of the vehicle detected by the motion detecting means.

The informing device thus constructed as previously mentioned according to the present invention can inform the vehicle occupants about the motion of the vehicle without failing to synchronize the image with the sound.

The informing device according to the present invention may further comprise acceleration detecting means for detecting an acceleration of the vehicle. The motion detecting means may be adapted to detect the motion of the vehicle on the basis of the acceleration detected by the acceleration detecting means.

The informing device thus constructed as previously mentioned according to the present invention can inform the vehicle occupants about the motion of the vehicle on the basis of the acceleration of the vehicle.

In the informing device according to the present invention, the motion detecting means may be adapted to detect the motion of the vehicle on the basis of a driver's action to drive the vehicle.

The informing device thus constructed as previously mentioned according to the present invention can allow the vehicle occupants to anticipate the change of the motion of the vehicle on the basis of the acceleration of the vehicle.

The informing device according to the present invention may further comprise navigation means for outputting support information to support a driver in driving to a destination through an optimum travel route. The motion detecting means may be adapted to detect the motion of the vehicle on the basis of the support information outputted by the navigation means.

The informing device thus constructed as previously mentioned according to the present invention can inform the vehicle occupants appropriately at the right time about the motion of the vehicle by obtaining detailed route information from the navigation means, and detecting the motion of the vehicle on the basis of the detailed route information.

The informing device according to the present invention may further comprise image taking means for taking an image of an object near or ahead of the vehicle. The motion detecting means may be adapted to detect the motion of the vehicle on the basis of the image taken by the image taking means.

The informing device thus constructed as previously mentioned according to the present invention can detect the motion of the vehicle more properly without being affected by clerical error or the like of road map information by using the image taken by the image taking means.

In the informing device according to the present invention, the controlling means may be adapted to control a position of a sound image to vehicle occupants on the basis of the motion of the vehicle detected by the motion detecting means.

The informing device thus constructed as previously mentioned according to the present invention can inform the vehicle occupants about the motion of the vehicle by controlling the position of the sound image to the vehicle occupants.

In the informing device according to the present invention, the controlling means may be adapted to change, on the basis of the motion of the vehicle detected by the motion detecting means, a period with which the sound is outputted.

The informing device thus constructed as previously mentioned according to the present invention can inform the vehicle occupants about the motion of the vehicle by changing a period with which the sound is outputted.

In the informing device according to the present invention, the controlling means may be adapted to control, in frequency, the sound on the basis of the motion of the vehicle detected by the motion detecting means.

The informing device thus constructed as previously mentioned according to the present invention can inform the vehicle occupants about the motion of the vehicle by controlling, in frequency, the sound.

In the informing device according to the present invention, the controlling means may be adapted to control, in amplitude, the sound on the basis of the motion of the vehicle detected by the motion detecting means.

The informing device thus constructed as previously mentioned according to the present invention can inform the vehicle occupants about the motion of the vehicle by controlling, in amplitude, the sound.

The informing device according to the present invention may further comprise setting means for setting control information to the controlling means.

The informing device thus constructed as previously mentioned according to the present invention can allow the vehicle occupants to change the control information if necessary.

In the informing device according to the present invention the controlling means may be adapted to have the outputting means output a specific sound by controlling the outputting means on the basis of the motion of the vehicle detected by the motion detecting means.

The informing device thus constructed as previously mentioned according to the present invention can inform the vehicle occupants appropriately at the right time about the change of the motion of the vehicle by using a sound. Even if the driver brakes hard suddenly, the vehicle occupants can anticipate and respond to the sudden change of the motion of the vehicle.

In the informing device according to the present invention, the controlling means may be adapted to have the outputting means output a specific image by controlling the outputting means on the basis of the motion of the vehicle detected by the motion detecting means.

The informing device thus constructed as previously mentioned according to the present invention can inform the vehicle occupants appropriately at the right time about the change of the motion of the vehicle by using an image together with a sound. If the driver brakes hard suddenly, the vehicle occupants can anticipate and respond to the sudden change of the motion of the vehicle.

### ADVANTAGEOUS EFFECT OF THE INVENTION

The present invention can provide an informing device for enhancing an effect of allowing vehicle occupants to enjoy comfortable driving.

### BRIEF DESCRIPTION OF THE DRAWINGS

FIG. **1** is a block diagram showing an embodiment of a motion sickness preventing apparatus according to the present invention.
FIG. **2** is a schematic top view showing an vehicle provided with the motion sickness preventing apparatus shown in FIG. **1**.
FIG. **3(a)** is a schematic top view showing a vehicle with the sound image located on the basis of one condition.
FIG. **3(b)** is a schematic top view showing a vehicle with the sound image located on the basis of another condition.
FIG. **3(c)** is a schematic top view showing the vehicle with the sound image located on the basis of further condition.
FIG. **4(a)** is a view showing a normal period with which the sounds are outputted from the motion sickness preventing apparatus shown in FIG. 1.
FIG. **4(b)** is a view showing a short period with which the sounds are outputted from the motion sickness preventing apparatus shown in FIG. 1.
FIG. **4(c)** is a view showing a long period with which the sounds are outputted from the motion sickness preventing apparatus shown in FIG. 1.
FIG. **5(a)** is a schematic top view showing the vehicle with the sounds controlled in amplitude, and outputted at one point in time.
FIG. **5(b)** is a schematic top view showing the vehicle with the sounds controlled in amplitude, and outputted at another point in time.
FIG. **6** is a flowchart showing an operation of the motion sickness preventing apparatus according to the embodiment of the present invention.

### EXPLANATION OF THE REFERENCE NUMERALS

- **10:**: motion sickness preventing apparatus (informing device)
- **11:**: acceleration detecting means
- **12:**: navigation means
- **13:**: image taking means
- **14:**: otion detecting means
- **15:**: image and sound producing means
- **16:**: image displaying means
- **17:**: sound outputting means
- **18:**: controlling means
- **19:**: setting means
- **20:**: vehicle (vehicle)

### DETAILED DESCRIPTION OF THE PREFERRED EMBODIMENT

One embodiment of the informing device according to the present invention will be described hereinafter with reference to accompanying drawings.

The following description will be directed to the constitution of the informing device according to the present invention.

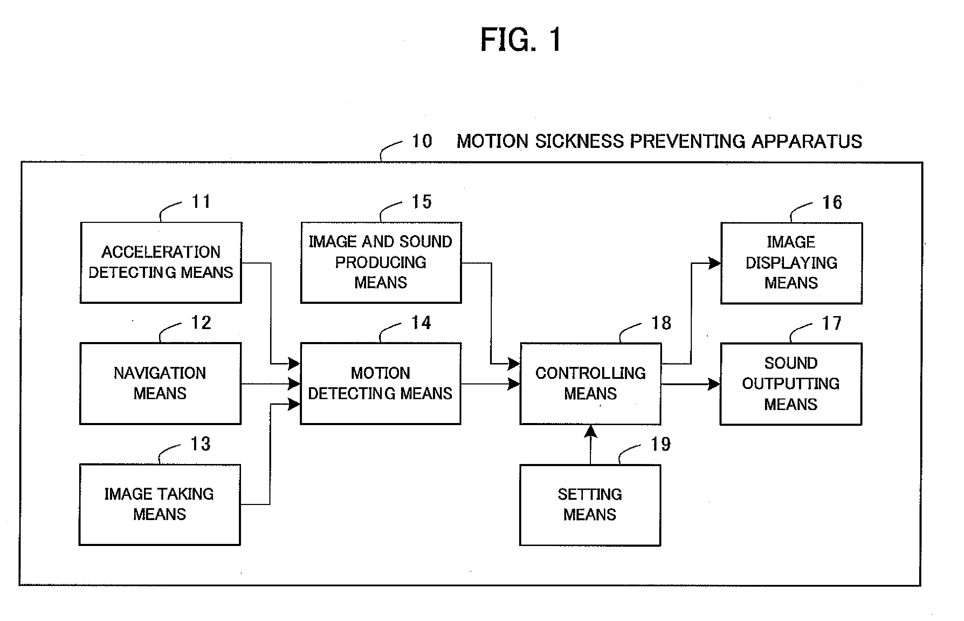

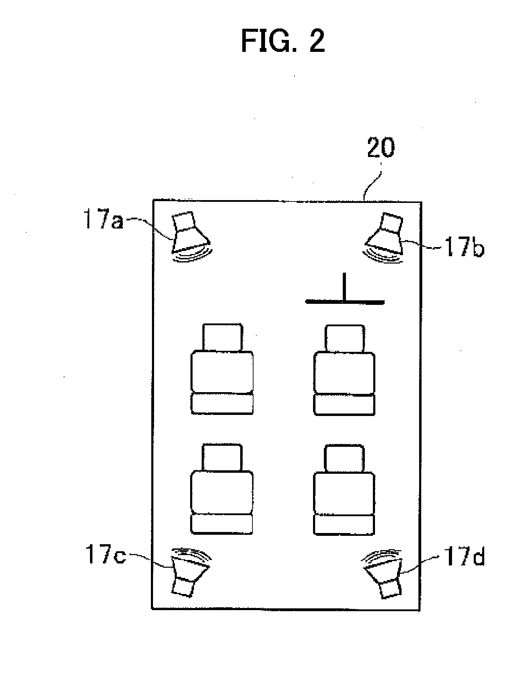

In this embodiment, the informing device according to the present invention is constituted as a motion sickness preventing apparatus. As shown in FIG. 1, the motion sickness preventing apparatus **10** comprises acceleration detecting means **11** for detecting an acceleration of a vehicle **20** (See FIG. **2),** navigation means **12** for outputting support information to support a driver in driving to a destination through an optimum travel route, image taking means **13** for taking, as outside environment information, an image of an object near or ahead of the vehicle **20,** motion detecting means **14** for detecting a motion of the vehicle **20,** image and sound producing means **15** for producing an image signal and a sound signal, image displaying means **16** for displaying an image on a screen in response to the image signal produced by the image and sound producing means **15,** sound outputting means **17** for outputting a sound in response to the sound signal produced by the image and sound producing means **15,** controlling means **18** for controlling the image displaying means **16** and the sound outputting means **17** on the basis of the motion of the vehicle **20** detected by the motion detecting means **14,** and setting means **19** for setting control information on the image displaying means **16** and the sound outputting means **17** to the controlling means **18.** The image displaying means **16** and the sound outputting means **17** are collectively constituted as outputting means for outputting an image with a sound.

Here, the acceleration detecting means **11** is constituted by, for example, at least one of a velocity sensor for detecting a velocity of the vehicle **20,** an acceleration sensor for detecting an acceleration of the vehicle **20,** and an angular velocity sensor for detecting an angular velocity of the vehicle **20.** The acceleration detecting means **11** detects an acceleration of the vehicle **20** in at least one of leftward/rightward, upward/downward, and forward/backward directions by using at least one of the velocity detected by the velocity sensor, the acceleration detected by the acceleration sensor, and the angular velocity detected by the angular velocity sensor. The motion detecting means **14** is adapted to detect, on the basis of the acceleration detected by the acceleration detecting means **11,** whether the vehicle **20** is turning to the right or the left, whether or not the vehicle 20 is being accelerated, or whether or not the vehicle **20** is being decelerated.

The navigation means **12** is constituted by a conventional navigation apparatus. The motion detecting means **14** detects, on the basis of the support information produced by the navigation means **12,** whether the vehicle **20** turns to the right or the left, whether or not the vehicle **20** is accelerated, or whether or not the vehicle **20** is decelerated. If the navigation means **12** is in an active state to support a driver in driving to a destination through an optimum travel route, the motion detecting means **14** receives information on the optimum travel route from the navigation means **12.** If, on the other hand, the navigation means **12** is in an inactive state not to support a driver in driving to a destination through an optimum travel route, the motion detecting means **14** receives, from the navigation means **12,** information on angles of right- or left-turn, an angle of each sloping road, a bank angle of each curve, a curvature, a road surface, a width, whether or not there is no traffic light, whether the vehicle **20** is traveling along a right turn lane or a left turn lane and the like.

The image taking means **13** is constituted by a camera. The motion detecting means **14** obtains, from one or more images taken by the camera, one or more features of a road ahead of the vehicle **20,** to determine, on the basis of the features of the road, whether the vehicle **20** turns to the right or the left, whether or not the vehicle **20** is accelerated, or whether or not the vehicle **20** is decelerated. Here, the information on one or more features of the road ahead of the vehicle **20** extracted from the images taken by the image taking means **13** is substantially the same the information received from the navigation means **12.**

The motion detecting means **14** is constituted by a central processing unit (CPU), a read only memory (ROM), and a random access memory (RAM). The motion detecting means **14** detects the motion of the vehicle **20** on the basis of driver's actions to drive the vehicle **20.** More specifically, the motion detecting means **14** detects, on the basis of at least one of operations of a steering wheel, an accelerator pedal, a brake pedal, a transmission shift lever, a direction indicator, a hazard indicator and the like, whether the vehicle **20** turns to the right or the left, whether or not the vehicle **20** is accelerated, or whether or not the vehicle **20** is decelerated.

The image and sound producing means **15** is constituted by an audio visual apparatus such as for example a television, a video player, a DVD player and the like.

The image displaying means **16** is constituted by a conventional display apparatus.

As shown in FIG. **2,** the sound outputting means **17** is constituted by a speaker unit **17a** located in front of a front passenger seat on the right, a speaker unit **17b** located in front of a driver seat on the left, a speaker unit **17c** located at the rear of a right rear passenger seat on the right, and a speaker unit **17d** located at the rear of a left rear passenger seat on the left. Needless to say, the present invention is not limited to the above-mentioned case. In other words, the sound outputting means **17** may be constituted by one or more speaker units.

The controlling means **18** shown in FIG. 1 is constituted by the CPU, the ROM, and the RAM.

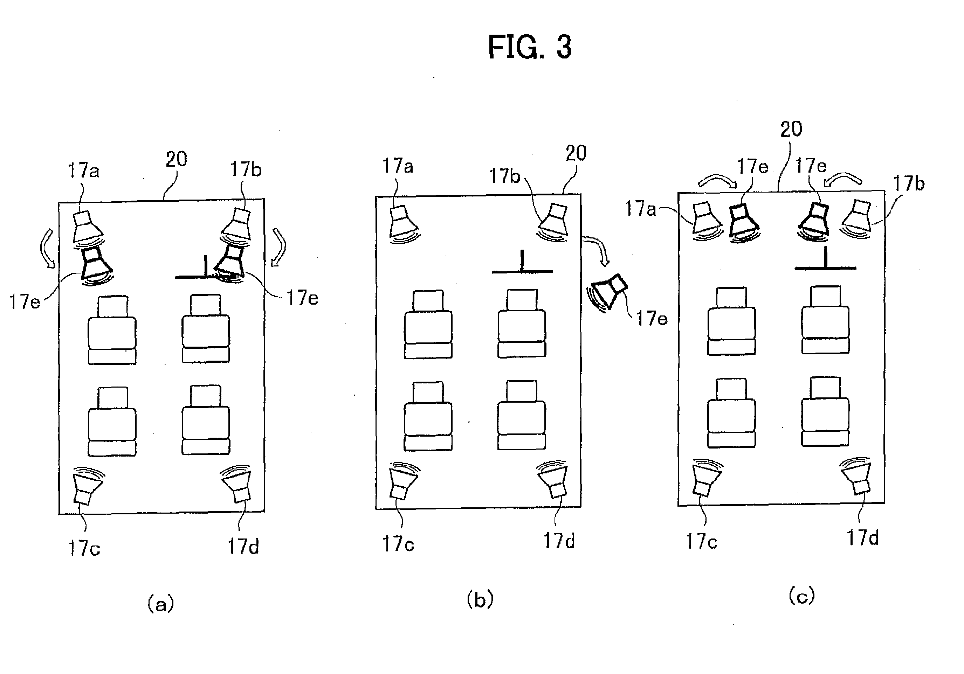

The controlling means **18** shifts one or more positions of sound images i.e. imaginary sound sources **17e** by controlling the sound outputting means **17** on the basis of the motion of the vehicle **20** detected by the motion detecting means **14.** As shown in FIG. **3(a),** the controlling means **18** controls the sound outputting means **17** to ensure that the imaginary sound sources **17e** are located in the vicinity of the vehicle occupants if the vehicle **20** is accelerated. On the other hand, the controlling means **18** controls the sound outputting means **17** to ensure that the imaginary sound sources **17e** are distanced from the vehicle occupants if the vehicle **20** is decelerated. The controlling means **18** controls the sound outputting means **17** to have the imaginary sound source **17e** shifted in the left side of the vehicle occupants if the vehicle **20** turns to the left. As shown in FIG. **3(b),** the controlling means **18** controls the sound outputting means **17** to have the imaginary sound source **17e** shifted in the right side of the vehicle occupants if the vehicle **20** turns to the right. Needless to say, the present invention is not limited to the above-mentioned case. As another example, the controlling means **18** may control the sound outputting means **17** to ensure that the imaginary sound sources are distanced from the vehicle occupants if the vehicle **20** is accelerated, and to ensure that the imaginary sound sources are located in the vicinity of the vehicle occupants if the vehicle **20** is decelerated. The controlling means **18** may control the sound outputting means **17** to have the imaginary sound sources shifted in an upward direction if the vehicle **20** is accelerated, and to have the imaginary sound sources shifted in a downward direction if the vehicle **20** is decelerated. The controlling means **18** may controls the sound outputting means **17** to have the imaginary sound sources shifted in a downward direction if the vehicle **20** is accelerated, and to have the imaginary sound sources shifted in an upward direction if the vehicle **20** is decelerated. As shown in FIG. 3(c), the controlling means **18** may control the sound outputting means **17** to have the imaginary sound source located in a narrow area defined in front of the vehicle occupants if the vehicle **20** is accelerated. The controlling means **18** may control the sound outputting means **17** to have the imaginary sound source located in a wide area defined in front of the vehicle occupants if the vehicle **20** is decelerated. In contrast, the controlling means **18** may control the sound outputting means **17** to have the imaginary sound source located in a wide area defined in front of the vehicle occupants if the vehicle **20** is accelerated. The controlling means **18** may control the sound outputting means **17** to have the imaginary sound source located in a narrow area defined in front of the vehicle occupants if the vehicle **20** is decelerated. In this embodiment, the positions of the imaginary sound sources are defined by the sounds outputted by the speaker units **17a** and **17b.** However, the positions of the imaginary sound sources may be defined by the sounds outputted by the speaker units **17c** and **17d.**

In this embodiment, the controlling means **18** controls the position of each imaginary sound source on the basis of a conventional method of locating the sound image in a designated area by using the speaker units **17a** to **17d.** This conventional method has been well known to those skilled in the art, and will not described hereinafter.

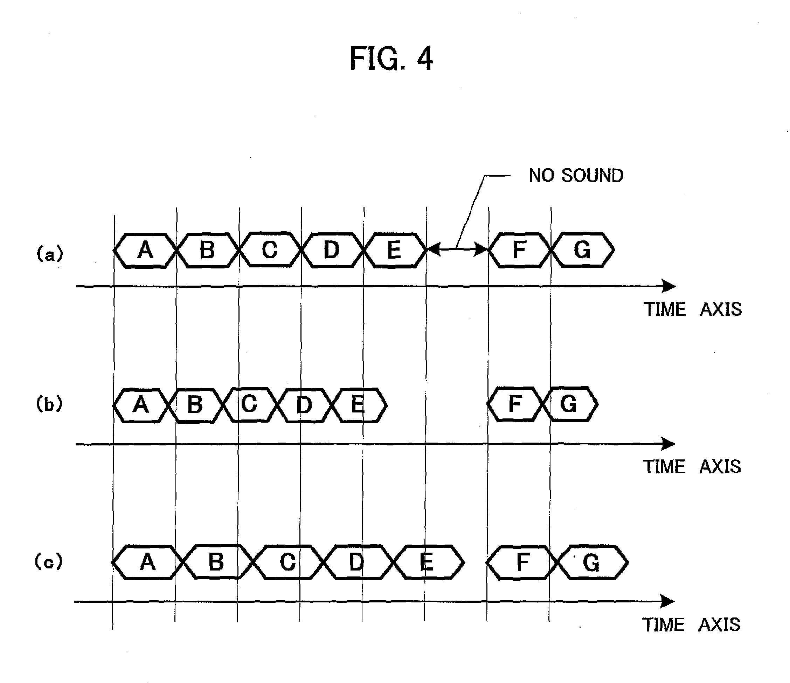

The controlling means **18** changes, on the basis of the motion of the vehicle **20** detected by the motion detecting means **14,** a period of time with which the sound is outputted by the sound outputting means **17.** As shown in FIG. **4(a),** the controlling means **18** allows the sound outputting means **17** to output the sound with a predetermined period of time if the degree of right- or left-turn, acceleration, and deceleration of the vehicle **20** does not exceed a predetermined threshold level. As shown in FIG. **4(b),** the controlling means **18** allows the sound outputting means **17** to output the sound with a shortened period of time when the degree of travel state of the vehicle **20** exceeds a predetermined threshold level. Needless to say, the present invention is not limited to the above-mentioned case. As shown in FIG. **4(c),** the controlling means **18** may allow the sound outputting means **17** to output the sound with a broadened period of time if the degree of right- or left-turn, acceleration, and deceleration of the vehicle **20** exceeds a predetermined threshold level.

The controlling means **18** changes, in frequency, the sounds to be outputted by the sound outputting means **17.** As an example, the controlling means **18** allows the sound outputting means **17** to heighten, in frequency, the sounds to be outputted by the sound outputting means **17** if the degree of right- or left-turn, acceleration, and deceleration of the vehicle **20** exceeds a predetermined threshold level. Needless to say, the present invention is not limited to the above-mentioned case. The controlling means **18** may be adapted to lessen, in frequency, the sounds to be outputted by the sound outputting means **17** if the degree of right- or left-turn, acceleration, and deceleration of the vehicle **20** exceeds a predetermined threshold level.

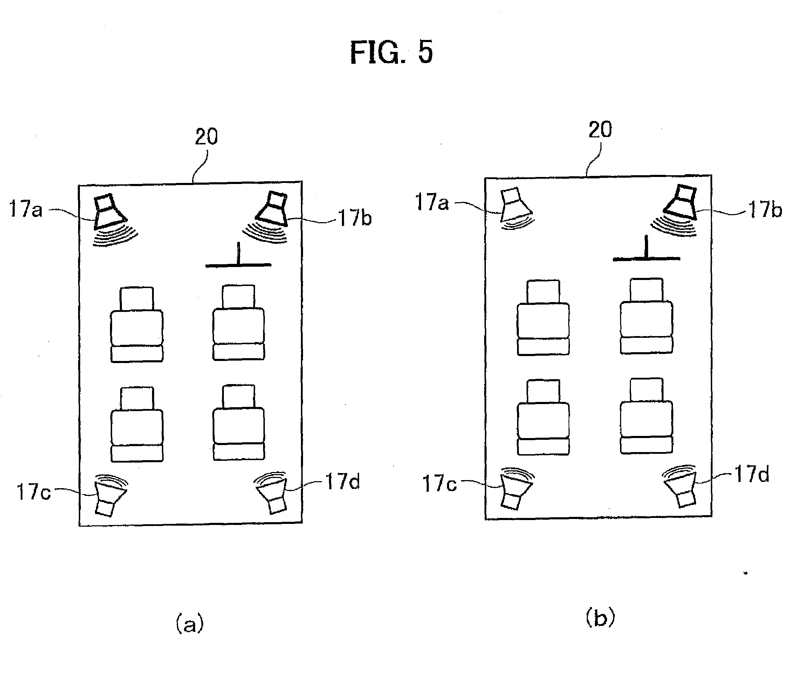

The controlling means **18** controls, in amplitude, the sound to be outputted by the sound outputting means **17** on the basis of the motion of the vehicle **20** detected by the motion detecting means **14.** As shown in FIG. **5(a),** the controlling means **18** turns up the volumes of the speaker units **17a** and **17b** if the driver accelerates the vehicle **20.** On the other hand, the controlling means **18** turns up the volumes of the speaker units **17c** and **17d** when the driver decelerates the vehicle **20.** As shown in FIG. **5(b),** the controlling means **18** turns up the volume of the speaker unit 17b if the driver has the vehicle **20** turn to the right. On the other hand, the controlling means 18 turns up the volume of the speaker unit **17c** if the driver has the vehicle **20** turn to the left. Needless to say, the present invention is not limited to the above-mentioned case. As another example, the controlling means 18 may be adapted to turn down the volume of the speaker unit **17b** if the driver has the vehicle **20** turn to the left, and to turn down the volume of the speaker unit **17a** if the driver has the vehicle **20** turn to the right. The controlling means **18** may turn up the volumes of the speaker units **17c** and **17d** if the driver accelerates the vehicle **20,** and may turn up the volumes of the speaker units **17a** and **17b** if the driver decelerates the vehicle **20.** As further example, the controlling means 18 may turn down the volumes of the speaker units **17a** and **17b** if the driver accelerates the vehicle **20,** and to turn down the volumes of the speaker units **17c** and **17d** if the driver decelerates the vehicle **20.** In contrast, the controlling means 18 may turn down the volumes of the speaker units **17c** and **17d** if the driver accelerates the vehicle **20,** and may turn down the volumes of the speaker units **17a** and **17b** if the driver decelerates the vehicle **20.**

If the controlling means **18** controls, on the basis of the motion of the vehicle **20** detected by the motion detecting means **14,** the sound to be outputted from the sound outputting means **17,** the controlling means synchronizes, with the sound to be outputted by the sound outputting means **17,** the image to be displayed by the image displaying means 16.

The setting means **19** is constituted by elements including, for example, a central processing unit (CPU), a random access memory (ROM), and a read only memory (ROM).

The following description will be directed to an operation of the motion sickness preventing apparatus **10** according to the embodiment of the present invention.

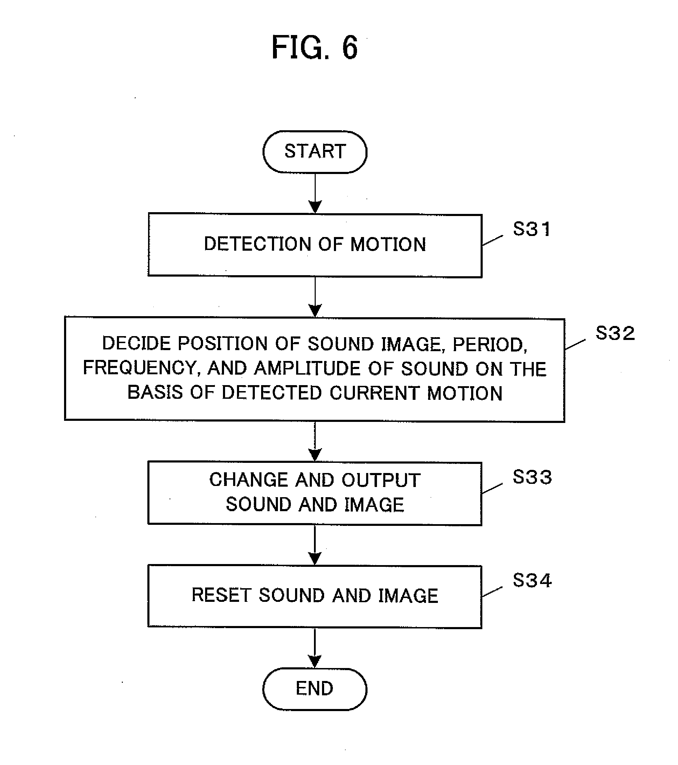

As shown in FIG. **6,** the current motion of the vehicle **20** is firstly detected by the motion detecting means **14** of the motion sickness preventing apparatus **10** (in the step S31).

In this embodiment, the motion detecting means **14** detects the degree of right and left turns of the vehicle **20** on the basis of the acceleration detected in the leftward or rightward direction by the acceleration detecting means **11** and a steering angle of the wheel steering of the vehicle **20,** or the shape of the road ahead of the vehicle **20** obtained from the road map information of the navigation means **12.** However, the motion detecting means **14** may detect the degree of left- or right-turn of the vehicle **20** on the basis of the shape of the road ahead of the vehicle **20** calculated from one or more images taken by the image taking means **13.**

In this embodiment, the motion detecting means **14** detecta the degree of acceleration and deceleration of the vehicle **20** on the basis of the acceleration detected in the forward or backward direction by the acceleration detecting means **11,** the degree of accelerator pedal, and speed information obtained from the navigation means **12.** However, the motion detecting means **14** may detect the degree of acceleration and deceleration of the vehicle **20** on the basis of the current speed of the vehicle **20** calculated from the current image of the road ahead of the vehicle **20** and the previous image of the road ahead of the vehicle **20.**

In the motion sickness preventing apparatus **10,** the controlling means **18** decides, on the basis of the travel state of the vehicle **20** detected in the step **S31,** a position of the imaginary sound source, a period of time, frequency, or amplitude of the sounds (in the step **S32).** The sound outputting means **17** outputs the sounds on the basis of the position of the imaginary sound source, the period of time, the frequency, or the amplitude decided by the controlling means **18** (in the step **S32**).

In the motion sickness preventing apparatus **10,** the controlling means **18** starts to compute an elapsed time after changing, in position, the imaginary sound source, and changing, in period of time, frequency, or amplitude, the sound to be outputted by the sound outputting means **17** on the basis of the travel state of the vehicle **20,** to reset, in position, the imaginary sound source, and to reset, in period of time, frequency, or amplitude, the sound to be outputted by the sound outputting means **17** if the elapsed time exceeds a designated level.

In the motion sickness preventing apparatus **10,** the setting means **19** allows an operator (i.e. each vehicle occupant) to set, to the controlling means **18,** information on whether or not to change, in position, each imaginary sound source, a direction in which each imaginary sound source is changed in position, a range within which each imaginary sound source is changed in position in each direction, a period of time with which each imaginary sound source is changed in position in each direction, a speed at which each imaginary sound source is changed in position in each direction, whether or not to change, in period of time, each sound to be outputted by the sound outputting means **17,** a range within which each sound is changed in period of time, a period of time with which each sound is changed in period of time, a speed at which each sound is changed in period of time, whether or not to change, in frequency, each sound to be outputted by the sound outputting means **17,** a range within which each sound is changed in frequency, a period of time with which each sound is changed in frequency, a speed at which each sound is changed in frequency, whether or not to change, in amplitude, each sound to be outputted by the sound outputting means **17,** a direction in which each sound is changed in amplitude, a range within which each sound is changed in amplitude in each direction, a period of time with which each sound is changed in amplitude in each direction, a speed at which each sound is changed in amplitude in each direction, and the like.

From the foregoing description, it will be understood that the motion sickness preventing apparatus **10** can inform the vehicle occupants about the motion of the vehicle **20** by using one or more sounds produced on the basis of the motion of the vehicle **20.** Accordingly, the vehicle occupants can anticipate changes of the motion of the vehicle **20** without observing carefully a motion of the vehicle **20** with their eyes, and keep pace with changes of the motion of the vehicle **20.** In other words, the informing device according to the present invention can enhance an effect of allowing the vehicle occupants to enjoy comfortable driving without allowing them to observe carefully a motion of the vehicle **20** with their eyes.

The motion sickness preventing apparatus **10** can inform the vehicle occupants about the motion of the vehicle **20,** by using one or more sounds produced on the basis of the motion of the vehicle **20,** while allowing the vehicle occupants to watch and listen to an audio-visual content such as TV program, or a video outputted from the DVD player.

If one or more sounds are outputted by the sound outputting means **17** on the basis of the motion of the vehicle **20,** or the vehicle occupants are informed about the motion of the vehicle **20,** the motion sickness preventing apparatus **10** can prevent an image to be watched as the audio-visual content from being deviated from a sound to be listened as the audio-visual content by synchronizing the image to be displayed by the image displaying means **16** with the sound to be outputted by the sound outputting means **17.** As another example, the motion sickness preventing apparatus **10** may allow the image displaying means **16** to output, at a fixed playback speed, an image to be watched as the audio-visual content, without synchronizing the image to be displayed by the image displaying means **16** with the sound to be outputted by the sound outputting means **17,** if the vehicle occupants are informed about the motion of the vehicle **20.** As further example, the motion sickness preventing apparatus **10** may not comprise image displaying means **16** if it is only necessary to allow the vehicle occupants to listen to music or other sound with no image.

The motion sickness preventing apparatus **10** can inform the vehicle occupants of the motion of the vehicle **20** by changing one or more positions of imaginary sound sources, a period with which the sound is outputted, amplitude, a frequency and the like. In this embodiment, the controlling means **18** changes all of the positions, the period, the frequency, and the amplitude on the basis of the motion of the vehicle detected by the motion detecting means **14.** As another example, the controlling means **18** may change one or more of the positions, the period, the frequency, and the amplitude on the basis of the motion of the vehicle detected by the motion detecting means **14.**

In the motion sickness preventing apparatus **10,** the motion detecting means **14** detects the motion of the vehicle **20** on the basis of the acceleration detected by the acceleration detecting means **11,** the support information outputted by the navigation means **12** and the like. As another example, the motion detecting means 14 may detect the motion of the vehicle **20** on the basis of any one of the acceleration detected by the acceleration detecting means **11,** the support information outputted by the navigation means **12** and the like.

The motion sickness preventing apparatus **10** may change one or more positions of imaginary sound sources, a period with which the sound is outputted, amplitude, a frequency and the like if the motion of the vehicle **20** i.e. the degree of each condition (in which the vehicle **20** is turning to the right or the left, in which the vehicle **20** is being accelerated, and in which the vehicle **20** is being decelerated) exceeds a threshold level.

The motion sickness preventing apparatus **10** may change, with accelerating speed, change one or more positions of imaginary sound sources, a period with which the sound is outputted, amplitude, a frequency and the like.

The motion sickness preventing apparatus **10** may change, with processing speed based on the degree of each condition, change one or more positions of imaginary sound sources, a period with which the sound is outputted, amplitude, a frequency and the like.

The motion sickness preventing apparatus **10** can inform the vehicle occupants about the motion of the vehicle **20** while allowing them to watch and listen to an audiovisual content such as TV program, or a video outputted from the DVD player. Needless to say, the present invention is not limited to the above-mentioned case. The motion sickness preventing apparatus **10** may record images corresponding to right-turning, left-turning, accelerating, and decelerating states of the vehicle **20** in preliminary step. The controlling means **18** may allow the image display means **16** to display an image selected from among the recorded images on the basis of the motion of the vehicle **20.** As another example, the motion sickness preventing apparatus **10** may record sounds indicative of right-turning, left-turning, accelerating, and decelerating states of the vehicle **20** in preliminary step. The controlling means **18** may allow the sound outputting means **17** to output a sound selected from the recorded sounds on the basis of the motion of the vehicle **20.** From the foregoing description, it will be understood that the motion sickness preventing apparatus **10** can inform the vehicle occupants about the travel state of the vehicle 20 clearly and rapidly even if the driver brakes hard. Methods of displaying an image selected from the recorded images, and outputting a sound selected from the recorded sounds have been well known to those skilled in the art, and will not described hereinafter.

In the motion sickness preventing apparatus **10,** the motion detecting means **14** calculates the degree of left- or right-turn, and acceleration and deceleration of the vehicle **20.** However, the motion detecting means **14** may be adapted to detect whether or not the vehicle is being accelerated, whether or not the vehicle is being decelerated, whether or not the vehicle is turning to the right, and whether or not the vehicle is turning to the left.

### INDUSTRIAL APPLICABILITY OF THE PRESENT INVENTION

As will be seen from the foregoing description, the informing device according to the present invention has an advantageous effect of allowing the vehicle occupants to enjoy comfortable driving without allowing them to observe the motion of the vehicle with their eyes, and useful as a motion sickness preventing apparatus for preventing the vehicle occupants from having motion sickness.

## Claims

1. An informing device, comprising:
motion detecting means for detecting a motion of a vehicle;
outputting means for outputting a sound; and
controlling means for controlling, on the basis of said motion of said vehicle detected by said motion detecting means, said sound to be outputted by said outputting means.

2. An informing device as set forth in claim 1, in which said outputting means is adapted to output a sound with an image.

3. An informing device as set forth in claim 2, in which said controlling means is adapted to allow said outputting means to synchronize said image with said sound by controlling said outputting means on the basis of said motion of said vehicle detected by said motion detecting means.

4. An informing device as set forth in claim 1, which further comprises acceleration detecting means for detecting an acceleration of said vehicle, and in which
said motion detecting means is adapted to detect said motion of said vehicle on the basis of said acceleration detected by said acceleration detecting means.

5. An informing device as set forth in claim 1, in which said motion detecting means is adapted to detect said motion of said vehicle on the basis of a driver's action to drive said vehicle.

6. An informing device as set forth in claim 1, which further comprises navigation means for outputting support information to support a driver in driving to a destination through an optimum travel route, and in which
said motion detecting means is adapted to detect said motion of said vehicle on the basis of said support information outputted by said navigation means.

7. An informing device as set forth in claim 1, which further comprises image taking means for taking an image of an object near or ahead of said vehicle, and in which
said motion detecting means is adapted to detect said motion of said vehicle on the basis of said image taken by said image taking means.

8. An informing device as set forth in claim 1, said controlling means is adapted to control a position of a sound image to vehicle occupants on the basis of said motion of said vehicle detected by said motion detecting means.

9. An informing device as set forth in claim 1, said controlling means is adapted to change, on the basis of said motion of said vehicle detected by said motion detecting means, a period with which the sound is outputted.

10. An informing device as set forth in claim 1, said controlling means is adapted to control, in frequency, said sound on the basis of said motion of said vehicle detected by said motion detecting means.

11. An informing device as set forth in claim 1, said controlling means is adapted to control, in amplitude, said sound on the basis of said motion of said vehicle detected by said motion detecting means.

12. An informing device as set forth in claim 1, which further comprises setting means for setting control information to said controlling means.

13. An informing device as set forth in any one of claims 8 to 12, said controlling means is adapted to have said outputting means output a specific sound by controlling said outputting means on the basis of said motion of said vehicle detected by said motion detecting means.

14. An informing device as set forth in claim 3, said controlling means is adapted to have said outputting means output a specific image by controlling said outputting means on the basis of said motion of said vehicle detected by said motion detecting means.

## Amended claims

### Amended claims under Art. 19.1 PCT

**1.** (original): An informing device, comprising:
motion detecting means for detecting a motion of a vehicle;
outputting means for outputting a sound; and
controlling means for controlling, on the basis of said motion of said vehicle detected by said motion detecting means, said sound to be outputted by said outputting means.

**2.** (original): An informing device as set forth in claim 1, in which said outputting means is adapted to output a sound with an image.

**3.** (original): An informing device as set forth in claim 2, in which said controlling means is adapted to allow said outputting means to synchronize said image with said sound by controlling said outputting means on the basis of said motion of said vehicle detected by said motion detecting means.

**4.** (original): An informing device as set forth in claim 1, which further comprises acceleration detecting means for detecting acceleration of said vehicle, and in which
said motion detecting means is adapted to detect said motion of said vehicle on the basis of said acceleration detected by said acceleration detecting means.

**5.** (original): An informing device as set forth in claim 1, in which said motion detecting means is adapted to detect said motion of said vehicle on the basis of a driver's action to drive said vehicle.

**6.** (original): An informing device as set forth in claim 1, which further comprises navigation means for outputting support information to support a driver in driving to a destination through an optimum travel route, and in which
said motion detecting means is adapted to detect said motion of said vehicle on the basis of said support information outputted by said navigation means.

**7.** (original): An informing device as set forth in claim 1, which further comprises image taking means for taking an image of an object near or ahead of said vehicle, and in which
said motion detecting means is adapted to detect said motion of said vehicle on the basis of said image taken by said image taking means.

**8.** (currently amended): An informing device as set forth in claim 3, said controlling means is adapted to have said outputting means output a specific image by controlling said outputting means on the basis of said motion of said vehicle detected by said motion detecting means.

**9.** (cancelled).
